# EUROPEAN PATENT APPLICATION

(11) **EP 1 269 945 A1**
(43) Date of publication of application: **02.01.2003**
(21) Application number: 01114143.9
(22) Date of filing: 11.06.2001
(51) Int. Cl.: A61F 5/443

(54) **Human waste collection bag comprising stress relief means**

(71) Applicant: THE PROCTER & GAMBLE COMPANY, Cincinnati, Ohio 45202 (US)
(72) Inventor: Hasegawa, Maya, Ashiya City (JP); Burns, Jay G., Kobe City (JP)
(74) Representative: Hirsch, Uwe Thomas M.H.

(57) **Abstract**

The present invention relates to human waste collection bags for babies, children or adults to be attached to a wearer. More particularly it relates to a human waste collection bag comprising a pouch (11) and an aperture (21) the aperture (21) being surrounded by a flange (12), the pouch (11) being at least partially joined to the flange (12), and the human waste collection bag comprising a stress relief means (50) to relief stress exerted by portions of the pouch (11) onto portions of the flange (12).

## Description

### Field of the invention

The present invention relates to human waste collection bags for babies, children or adults to be attached to a wearer. More particularly it relates to a stress relief means (50) for such a collection bag.

### Background of the invention

Human waste collection bags are known articles of manufacture that are designed to be worn principally by incontinence sufferers and in particular by bedridden patients. Such human waste collection bags are attached to the perianal area or uro-genital area of the wearer and are intended to entrap and immediately contain faecal material, urine and other bodily discharges.

Faecal collection bags as they are mostly known today are constituted of a relatively long and narrow tube, at one extremity of which is positioned the aperture and the attachment device, which can be adhesive.

Because of their shape and dimensions, such devices can twist around the thighs of the wearers and lead to folds and kinks in the devices themselves. Such features naturally affect the storage capacity of the device and importantly may result in unintentional detachment of the device from the wearer due to pulling forces acting upon the pouch (11) leading to undesirable and distressing consequences both for the wearer and carer.

Such bags are disclosed in e. g. the following documents:

US 3,577,989, which details a disposable elimination-trapping bag for incontinence sufferers including a container member having an open-top portion, and a flange secured to the container member around the open-top portion. The container member includes two opposed side members, preferably substantially identical, and of a generally rectangular configuration, joined together along common edges. US 4,784,656, which describes a receptacle for collecting faecal matter from incontinence sufferers. The faecal collector comprises a gasket, conduit means or a cylinder and a receptacle; the receptacle and conduit means are each formed from two sheets of thermoplastic film that are heat sealed along their side edges, respectively. GB 2 152 387, which teaches a faecal collector for incontinence sufferers comprising a collection bag and a ring. The faecal collector comprises a pair of panels of thermoplastic sheet material joined at their margins to define an elongate bag having an opening at one end. In a preferred embodiment, the collection bag is formed from a single sheet of odour-barrier thermoplastic film folded along a vertical midline to provide a pair of continuous panels. SE 8 104 934, which discloses an oblong bag made from a thin, flexible and fluid tight material. The collecting bag comprises an inlet portion and a bottom portion at an angle of 120 degrees to the longitudinal direction of the inlet portion. The bag is so designed as to enable it to assume an advantageous position along the thigh of the person when in use.

The wearing conditions will depend on the nature of the wearer; when the wearer is active, such as a baby or a child, or an incontinent adult not being bedridden, the wearing conditions for the bag will become much more stressed and the risk of detachment of the bag will increase substantially, due to the movement of the wearer and pressure from the wearer's body. Pressure in particular upon the flange may not only result from outer forces acting directly upon the human waste collection bag when a wearer is active, but also from faecal material, once excreted and contained in the bag. Such pressure, in particular the flange, may result in the unintentional detachment of the bag.

It has now been found that the above drawbacks will be substantially alleviated if the bags are provided with stress relief means (50), thus allowing utilisation of the bags for babies, children and active adult incontinents in addition to bedridden adult incontinents.

### Summary of the invention

The present invention relates to human waste collection bags for babies, children or adults to be attached to a wearer. More particularly it relates to a human waste collection bag comprising a pouch (11) and an aperture (21) the aperture (21) being surrounded by a flange (12), the pouch (11) being at least partially joined to the flange (12), and the human waste collection bag comprising a stress relief means (50) to relief stress exerted by portions of the pouch (11) onto portions of the flange (12).

### Brief description of the drawings

It is believed that the invention will be better understood from the foregoing description in conjunction with the accompanying drawings in which:
Figure 1 is a perspective view of a preferred embodiment of a faecal collection bag.
Figure 2 is a perspective view of a preferred embodiment of a faecal collection bag comprising a portion of a stretchable material (52) as a stress relief means (50).
Figure 3 is a perspective view of a preferred embodiment of a faecal collection bag comprising an orifice (54) as a stress relief means (50), the orifice (54) being covered by a curtain (56).
Figure 4 is a perspective view of a preferred embodiment of a faecal collection bag comprising a portion comprising extra material (58) as a stress relief means (50).

### Detailed description of the invention

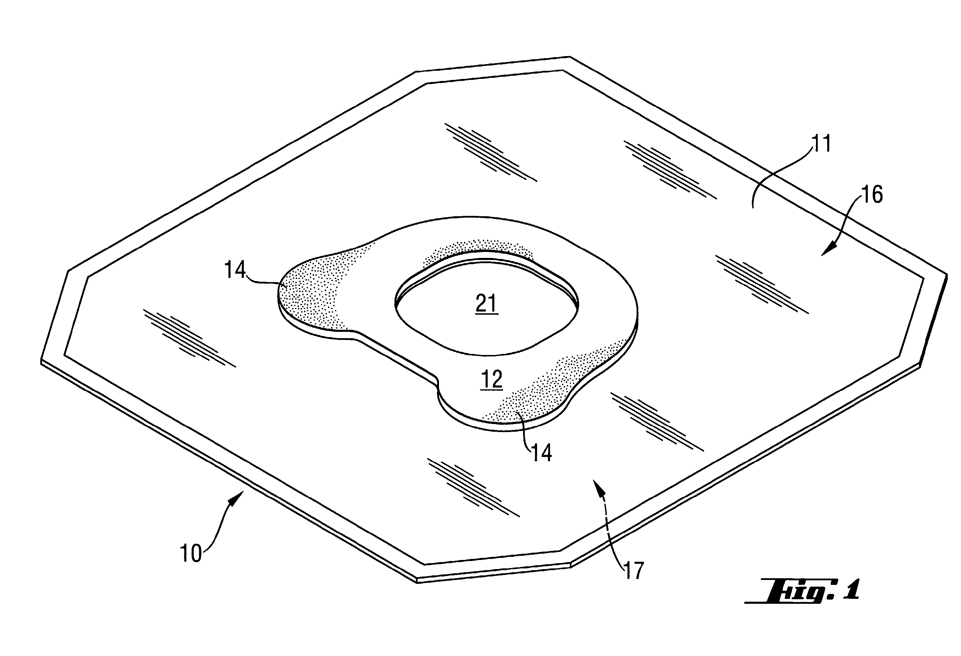

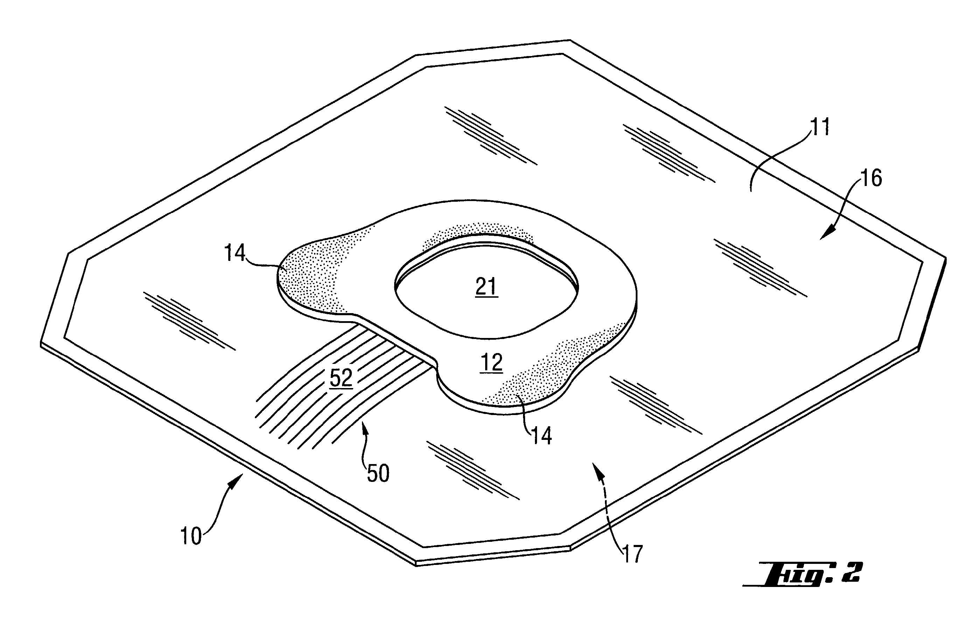

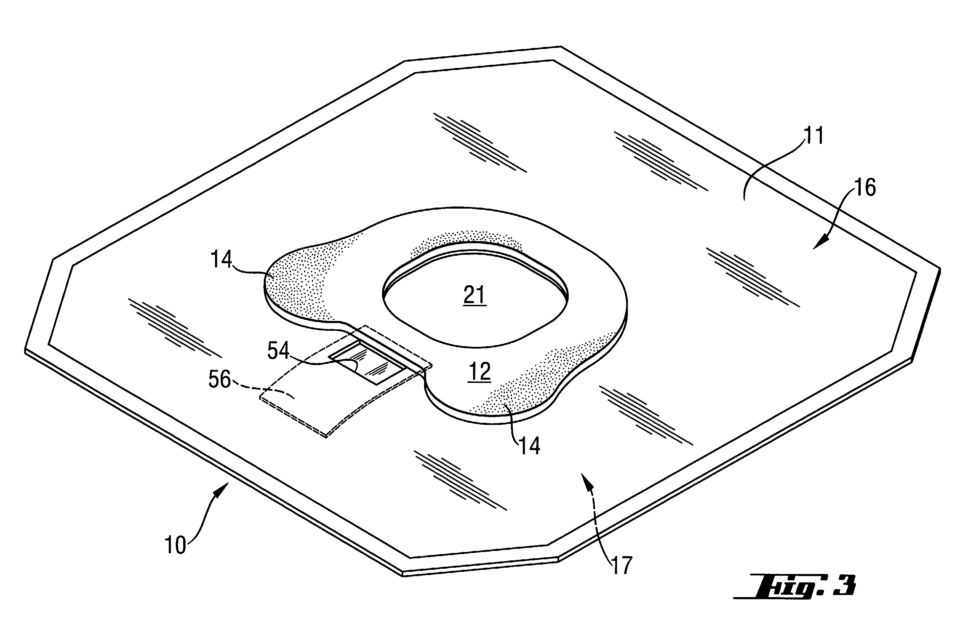

Human waste collection bags comprise faecal collection bags (10) and urine collection bags. Both types of collection bags can comprise similar or alike components and materials and are therefore described together. Figure 1 shows a faecal collection bag (10) according the present invention. Faecal collection bags (10) are designed for attachment to the anal area and mainly used for collecting faeces, whereas urine collection bags are attached to the urinary duct and mainly used for collecting urine. The present invention is preferably applied to faecal collection bags (10) and urine collection bags , but is also applicable to ostomy devices. All of the above human waste collection bags are preferably designed for single use and disposal thereafter.

Typically human waste collection bags comprise a pouch (11) having an aperture (21) and a flange (12) surrounding the aperture for preferably adhesive attachment to the perianal area of a wearer as visible from Figure 1. Any human waste collection bag known in the art can be provided according to the present invention.

The pouch (11) as used herein is a flexible receptacle for the containment of excreted faecal matter or urine. The pouch (11) is designed to safely contain any entrapped material, typically it will be liquid impermeable, yet it may be breathable. The pouch (11) is designed of sufficient strength to withstand rupture in use, also when pressure on the pouch (11) is exerted in typical wearing conditions, such as sitting.

The flange (12) is attached to the pouch (11) according to any means known to the man skilled in the art which may provide permanent or releasable attachment. Preferably however, the flange is attached to the pouch (11) by adhesive. Typically, the pouch (11) will be attached to the flange, towards the outer periphery of flange so as not to cause any obstruction for the entering faecal matter or urine.

When conceiving the present invention it has been found that the occurrence of leakage via unreliable attachment of the flange (12) to the body of the wearer and at the same the occurrences of a detachment of the collection bag can be significantly reduced if a stress relief means (50) is provided for the human waste collection bag. A stress relief means (50) according to the present invention can take a variety of forms, to be described hereinafter, but essentially serves to relief the stress exerted by the pouch (11) onto the flange (12) when the collection bag is worn. Such stress stems primarily either from faecal material contained in the pouch (11) when such material is subjected to pressure or stems from forces acting upon the pouch (11) form outside, especially when a wearer is active.

It has been found that such a stress relief means (50) is most efficient when provided in the wearer facing portion (16), as defined below. Most preferably at least a portion of the stress relief means (50) is joined to, coextensive of or comprised by a portion of the flange (12).

It has further been found that the stress relief means (50) is most efficient when being provided to relief stress exerted onto the perineal area of the flange (12).

The flange (12) of the human waste collection bag will generally follow the contour of the perineum and hence assume essentially an inverted V-shape. It has further been found that the most severe forces act upon the summit of this inverted V, hence the center of the perineum. Therefore according to the present invention the stress relief means (50) is preferably located adjacent to the central perineal area of the flange (12).

Without wishing to be bound by theory it is believed that the stress relief means (50) provided in the central perineal area of the flange (12) avoids that forces occurring in a direction generally perpendicular to the perineum act as perpendicular forces onto the flange (12), but those forces act as shear forces onto the sides of the flange outside the perineum, ie in the buttocks area. Many adhesives, eg hydrogel adhesives, have a much greater resistance to shear forces than to forces acting perpendicular on the adhesive layer.

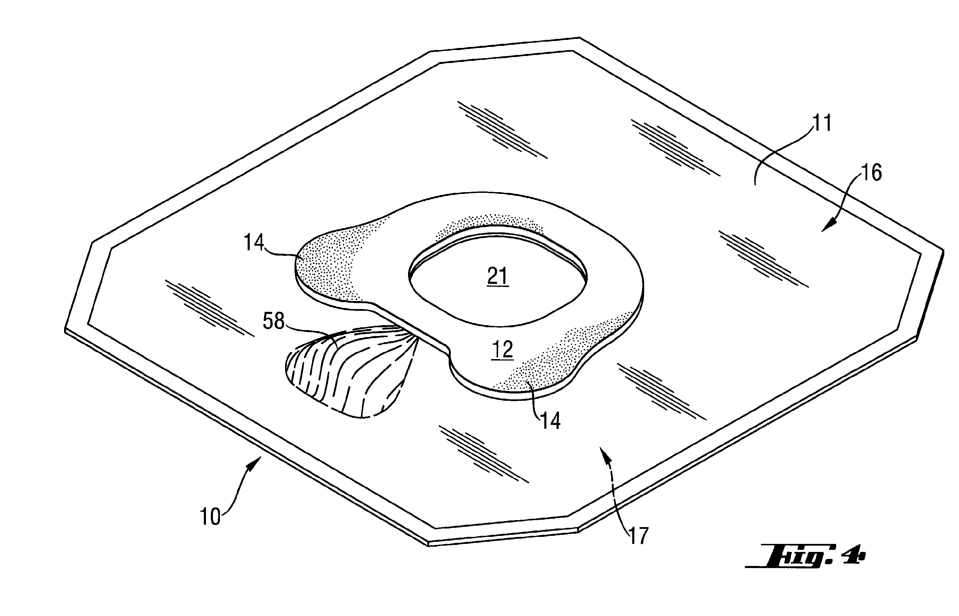

In one preferred embodiment of the present invention which is shown in Figure 2 the stress relief means (50) comprises a stretchable material in the area adjacent to the central perineal area; the stretchable material may be comprised by the pouch (11). Less stretchable material is provided, and typically comprised by the pouch (11), in the areas adjacent to the central perineal area.

As used herein, the term "stretchable material" is to be understood as referring to a material having a strain modulus of less than 1000 grams per percent strain per cm width. Preferred stretchable materials and preferred stress relief means (50) will afford a strain modulus in the range of 1 to 1000, preferably 5 to 500, more preferably 10 to 100 and most preferably 15 to 30 grams per percent strain per cm width. Materials having a strain modulus of more than 1000 grams per percent strain per cm width, and typically more than 1500 grams per percent strain per cm width are herein referred to as "less stretchable materials".

In another preferred embodiment the stress relief means (50) may comprise an orifice (54). This orifice (54) is not primarily provided as a passage way, but the orifice (54) will hinder the transmission of any forces onto the critical central perineal area. In one preferred embodiment of the present invention the pouch (11) is partially not joined to said flange (12) such that an orifice (54) is provided.

It has been found that faecal material will typically not leak through an orifice provided underneath the flange (12) and adjacent to the central perineal flange area. However, in a preferred embodiment the orifice (54) is covered by a curtain (56), eg a flap of pouch material, which is attached eg to the flange (12) and preferably only to the flange (12), such that no forces are transmitted and which provides a sealing for the orifice (56). As a secondary function the orifice (54) can also serve as an air inlet to facilitate the unfolding of the pouch (11) before use and as an air or gas outlet eg to take account of flatulence. Such an preferred embodiment is shown in Figure 3.

In a highly preferred embodiment of the present invention the stress relief means (50) is an integral part of the pouch (11), see Figure 4. A stress relief means (50) can be provided by providing extra material (58) adjacent to the flange (12) and preferably the central perineal portion of the flange (12). Most preferably the extra material (58) forms part of the pouch (11), such that the stress relief means (50) comprises a portion of the pouch (11) in which extra material (58) is provided between the flange (12) and the distal end of the pouch (11) as compared to adjacent portions of the pouch (58).

As used herein, the term "extra material (58)" denotes material comprised by the pouch (11) which does not transmit any force towards the flange (12) when such forces acts upon the pouch (11).

The pouch will comprise a wearer facing portion (16) and a garment facing portion (17). As used herein the wearer facing portion (16) is to be understood as the portion of the pouch (11) generally facing towards the wearer and the garment facing portion (17) is to be understood as the portion of the pouch (11) generally facing the garment of the wearer. When a diaper is worn in addition to a human waste collection bag the garment facing portion (17) will be in contact with the diaper.

Depending on the shape of the pouch (11) required, the pouch (11) may be provided from a unitary piece of material or a number of separate pieces of material, which may be identical or different and which are sealed at their respective peripheries. The preferred shape of the pouch (11) depends in particular on the intended use thereof, i.e. whether the device is intended for bedridden patients or active patients suffering from incontinence or requiring an artificial bowel or for infants.

The pouch (11) described herein preferably have a wearer facing portion (16) and a garment facing portion (17), which both comprise separate pieces of material. The wearer facing portion (16) and the garment facing portion (17) are sealed at the periphery of the pouch (11), thus creating a bag peripheral rim. The wearer facing portion (16) and the garment facing portion (17) may each independently comprise more than one section of material. Preferably the garment facing portion (17) comprises only one section of material; most preferably also the wearer facing portion (16) comprises only one section of material.

The wearer facing portion (16), the garment facing portion (17) and the pieces of material comprised by either of these portions are secured to each other by means known to the man skilled in the art, such as adhesive, thermobonding or pressure bonding in order to provide the desired bag configuration. The rim, at which the wearer facing portion (16) and the garment facing portion (17) are sealed together, may be provided inside the pouch (11) rather than outside the pouch (11), thus being coextensive with the inner surface of the pouch (11) rather than with the outer surface of the pouch (11).

Preferably the shape of the pouch should be chosen such that the surface area of the garment facing portion (17) is greater than the surface area of the wearer facing portion (16). Preferably the surface area of the garment facing portion (17) is at least 10%, preferably 25%, yet preferably 50%, yet more preferably 75% greater than the surface area of the wearer facing portion (16). When the garment facing portion (17) comprises folds (19) as in one preferred embodiment of the present invention the surface area of the garment facing portion (17) is to be understood as the surface area when the garment facing portion (17) is unfolded, ie. corresponds to the surface area of the sheet of material used to provide the garment facing portion (17).

The term "longitudinal" as used with regard to the human waste collection bag is to be understood as follows: the plane which bisects an upright standing wearer in a left and a right half shall comprise the longitudinal direction of the human waste collection bag. The longitudinal axis is typically an axis of symmetry of the pouch (11).

In another important aspect of the present invention the garment facing portion (17) should comprise a malleable material.

Pouches (11) according to the present invention can have a variety of shapes. One preferred shape is shown in Figures 1; in this preferred embodiment the wearer facing portion (16) is of an essentially square shape, the corner areas of the corresponding square, however, being "cut-off', such that an irregular octogon is obtained. Any essentially rectangular shape, much preferably with "cut-off' or flattened corners, is preferred.

When an faecal collection bag (10) for an incontinent adult is provided the pouch (11) as shown in Fig. 1 should measure from 10 cm to 30 cm, preferably 15 cm to 25 cm in the longitudinal direction and from 10 cm to 30 cm, preferably 15 cm to 25 cm in the transversal direction.

The pouch may comprise only a single orifice, the aperture (21), but may also comprise additional orifices or valves. For example, an air and gas outlet means, in the form of an orifice or a valve can be used to allow for the outlet of flatulence gases or the like. Moreover, an air inlet means, eg in the form of a hole in the garment facing portion (17), has proven useful to facilite the quick unfolding of the pouch (11). If the stress relief means (50) is provided in the form of an orifice (54), the orifice (54) can fulfil the additional functions of serving as an air and gas inlet and outlet means.

The pouch material, and hence the material of the wearer facing portion (16) and the material of the garment facing portion (17), can comprise one or multiple layers, preferably two or three layers. The layer on the inside of the pouch (11), which will typically at least partially come in contact with faecal material or urine is called the inner layer. The outermost layer of the pouch (11), which will typically at least partially come in contact with the skin to the wearer and the garments of the wearer, is called the outer layer.

The layers of the pouch (11) material may comprise any material, preferably so that the pouch (11) is liquid impervious. The layers may in particular comprise any material such as non-wovens or films. In a preferred embodiment of the present invention a laminate may be formed from a non-woven layer and a film. The laminate can be formed by means known to the man skilled in the art.

Any non-woven layer can comprise felt fabrics, spunlaced fabrics, fluid jet entangled fabrics, air-laid fabrics, wet-laid fabrics, dry-laid fabrics, melt-blown fabrics, staple fibre carding fabrics, spunbonded fabrics, stitch-bonded fabrics, apertured fabrics, combinations of the above or the like.

Suitable film materials for any of said layers preferably comprise a thermoplastic material. The thermoplastic material can be selected from among all types of hot-melt adhesives, polyolefins especially polyethylene, polypropylene, amorphous polyolefins, and the like; material containing meltable components comprising fibres or polymeric binders including natural fibres such as cellulose - wood pulp, cotton, jute, hemp; synthetic fibres such as fibreglass, rayon, polyester, polyolefin, acrylic, polyamid, aramid, polytetrafluroethylene metal, polyimide; binders such as bicomponent high melt/low melt polymer, copolymer polyester, polyvinyl chloride, polyvinyl acetate/chloride copolymer, copolymer polyamide, materials comprising blends wherein some of the constituent materials are not meltable; air and vapour permeable materials including microporous films such as those supplied by EXXON Chemical Co., III, US under the designation EXXAIRE or those supplied by Mitsui Toatsu Co., Japan under the designation ESPOIR NO; and monolithic breathable materials such as Hytrel™ available from DuPont and Pebax™ available from ELF Atochem, France.

In a preferred embodiment a film, which is comprised in any layer, is preferably permeable to gases such as air and to vapour such as water vapour in order to avoid the problem of entrapment and condensation of moisture vapour given off by the body of the wearer and thus, the hot, clammy and uncomfortable conditions after a short period of use.

The outer layer of the pouch (11) material may comprise a non-woven layer. Such material layers present an uneven surface to the skin of the wearer and thus reduce significantly the problem of occlusion and greatly improve skin healthiness.

In one preferred embodiment of the present invention the pouch (11) material comprises two layers. Preferably the outer layer comprises a non-woven layer and the inner layer comprises a film.

In yet another preferred embodiment of the present invention, the pouch (11) material comprises three layers, preferably one film and two non-woven layers. In an even more preferable embodiment the film is interposed between the two non-woven layers. This sequence of layers results in a closed fibrous structure, which has a particularly pleasing sensation on contact with the skin of the wearer. In yet another preferred embodiment the inner layer comprises a film and the other two layers comprise non-wovens.

The non-woven layer or the non-woven layers comprised by the pouch (11) material may be hydrophobic or hydrophilic. If the pouch (11) material does not comprise a film layer, preferably at least one non-woven layer is hydrophobic. As a consequence, fluid penetration is resisted through the wearer facing portion (16) and the garment facing portion (17) of the human waste collection bag. If the pouch (11) material comprises a film or a hydrophobic non-woven layer, further non-woven layers may be hydrophilic.

Typically, the non-woven layer is treated with a surface active material, such as a fluorchemical or other hydrophobic finishings, to provide the requisite hydrophobicity. The non-woven layer, however, may equally be treated with coatings of liquid impervious materials such as hot-melt adhesives or coatings of silicone or other hydrophobic compounds such as rubbers and vegetable and mineral waxes or it may be physically treated using nano-particulates or plasma coating techniques, for example.

The non-woven layer can also be treated with agents to improve the tactile perceivable softness of the wearer facing portion (16) and the garment facing portion (17). The agents include but are not limited to vegetable, animal or synthetic oils, silicone oils and the like. The presence of these agents are known to impart a silky or flannel-like feel to the non-woven layer without rendering it greasy or oily to the tactile sense of the wearer. Additionally, surfactant material, including anionic, cationic, non-ionic and amphoteric surfactants, may be added to further enhance softness and surface smoothness.

Furthermore, the non-woven layer may be impregnated with a lotion to provide desirable therapeutic or protective coating lotion benefits. The lotion coating on the wearer facing portion (16) and the garment facing portion (17) is transferable to the skin of the wearer by normal contact and wearer motion and/or body heat. Generally, mineral oil in the form of a lotion is recognised as being effective in imparting a soothing, protective coating to the skin of the wearer. It is also possible to impregnate the non-woven layer with a solid oil phase of cream formulation or to incorporate into the non-woven layer an array of pressure- or thermal- or hydrorupturable capsules containing for example, baby oil.

In one embodiment of the present invention the pouch (11) may contain absorbent material. The absorbent material may comprise any absorbent material which is capable of absorbing and retaining liquids. The absorbent material may comprise a wide variety of liquid-absorbent materials commonly used in disposable diapers and other absorbent articles such as comminuted wood pulp, which is generally referred to as airfelt. Examples of other suitable absorbent materials include creped cellulose wadding; meltblown polymers, including coform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

The absorbent material may be positioned in the pouch (11) in any suitable manner. For example, the absorbent material may be loosely arranged within the pouch (11) or may be secured to the inner surface of the pouch (11). Any known techniques for securing absorbent material to nonwoven and film substrates may be used to secure the absorbent material to the inner surface of the pouch (11). The absorbent material may also be arranged to have any desired shape or configuration (e.g., rectangular, oval, circular, etc.).

The pouch (11) is provided with an aperture (21) whereby faecal matter or urine is received from the body prior to storage within the pouch (11) cavity. The aperture (21) is surrounded by a flange (12) and may be provided in any shape or size, such as circular, oblong, heart shaped and may be symmetrical or asymmetrical, preferably the aperture has an oblong configuration either in the longitudinal or in the transversal direction.

The flange may be provided in any size depending on the wearer group for which the device is intended. Similarly the flange may be provided in any shape and preferably has a symmetrical shape preferably comprising a plurality of lobes.

The flange comprises a garment facing portion and a wearer facing portion. In an preferred embodiment these are two large, substantially flat surfaces, however, the flange (12) may also comprise projections, a front projection and/or a rear projection, in case of a faecal collection bag (10) designed to fit the perineal and/or coccygeal area of the wearer and in case of a urine collection bag designed to fit the genital and/or perineal area.

The flange (12) should be made of soft, flexible and malleable material to allow easy placement of the flange (12) to the perianal or uro-genital area. Typical materials include nonwoven materials, wovens, open celled thermoplastic foams, closed-cell thermoplastic foams, composites of open celled foams and stretch nonwoven, and films. A closed-cell foam of polyethylene has been found effective, but more preferably an open celled polyurethane foam is used. Preferably, such foams have a thickness within the general range of 0.1 to 5 millimetres and a density of 5 to 250 g/l more preferably 50 g/l. Other thermoplastic foam materials, or other suitable plastics sheet materials having the described properties of such foams (i.e., softness, pliability, stretchability, and contractability) might also be used. Preferably, the material of garment facing portion of the flange (12) may extend into the defined aperture area so as to form a skirt or flap of material which prevents unintentional adhesion of the surface edges of the flange (12) defining the aperture (21) to one another during use.

The human waste collection bag further comprises an attachment means to secure the device to the wearer. Such means include straps and more preferably comprises a body-compatible pressure sensitive adhesive applied to the wearer facing portion of the flange (12).

The adhesive is preferably covered with a release means (not shown) in order to protect the adhesive, such as siliconised paper. The adhesive can cover the entire wearer facing portion of the flange (12), more preferably the flange (12) has at least one, preferably two to six non-adhesive portions. These portions may be adhesive free or may contain inactivated or covered adhesives. As is evident from Figure 1, the adhesive is in one preferred embodiment not applied to the entire wearer facing portion of the flange (12), so as to provide lobes on either side of the flange (12) which are non-adhesive and can thereby serve to facilitate placement and removal of the device whilst avoiding contact with the adhesive. These lobes are however preferably also covered by the release means. Before application of the human waste collection bag to the skin of the wearer, the release means if present is removed.

Any medically approved water resistant pressure sensitive adhesive may be used to attach the device to the perianal or uro-genital area of the wearer, such as hydrocolloid adhesives and hydrogel adhesives. Particularly effective adhesives in providing the desired adhesive properties to secure the flange to the skin of the wearer at the sensitive perianal area, whilst allowing for relatively painless application and removal, are formed from crosslinking polymers with a plastisicer to form a 3-dimensional matrix.

The adhesive can be applied to the wearer facing portion of the flange (12) by any means known in the art such as slot coating, spiral, or bead application or printing. Typically the adhesive is applied at a basis weight of from 20g/m² to 2500g/m², more preferably from 500g/m² to 2000g/m² most preferably from 700g/m² to 1500g/m² depending on the end use envisioned. For example, for human waste collection bags to be used for babies the amount of adhesive may be less than for human waste collection bags designed for active adult incontinence sufferers.

In a preferred embodiment the human waste collection bag is provided in a particular configuration prior to use and, preferably, provided together with an applicator.

In one such configuration, the flange (12) is folded along the longitudinal axis to allow easier placement of the flange (12) in-between the buttocks of a wearer and the pouch (11) is preferably folded, which provides numerous advantages. For example, folding of the pouch (11) allows a smaller packaging format of the human waste collection bag, thus reducing the costs for transport and packaging material. Furthermore, the handling of the human waste collection bag is more convenient if the pouch (11) is folded, since the pouch (11) may otherwise cover parts of the applicator e.g. the handling portion. The pouch (11), when not folded, may also cover parts of the body of the wearer, so that the person placing the human waste collection bag cannot sufficiently visually control the placing.

In another preferred prior to use configuration the pouch (11) is provided unfolded. This configuration is particularly suitable for relatively small pouches (11), which can be provided according to the present invention. A relatively small outer contour of the pouch (11) is best achieved, when folds (19) are provided in the garment facing portion (17). Being able to provide the pouch (11) in an unfolded condition allows easier manufacturing and packaging.

Most preferably the applicator and the human waste collection bag in combination are provided in a particular configuration prior to use. This allows ready application of the human waste collection bag with only a few handling steps, since there is no need to have separate items at hand (the applicator and the human waste collection bag) and to position the applicator on the appropriate areas of the human waste collection bag for application.

The applicator may be provided with a means to hold the applicator and the human waste collection bag together. This means will typically also ensure the correct positioning of the applicator relative to the human waste collection bag and help to maintain the folded configuration of the pouch (11), if the bag is folded. Such a means preferably are provided in the form of any string or band, which may be provided in form of a closed loop, such as a rubber band. Such a means may also be provided in form of a clamp or a clip, made from any suitable material such as plastic or metal.

## Claims

1. A human waste collection bag comprising a pouch (11) and an aperture (21) the aperture (21) being surrounded by a flange (12), said pouch (11) being at least partially joined to said flange (12), **characterised in that** said human waste collection bag comprises a stress relief means (50) to relief stress exerted by portions of said pouch (11) onto portions of said flange (12).

2. A human waste collection bag according to claim 1, wherein the pouch (11) comprises a wearer facing portion (16) and a garment facing portion (17) and said stress relief means (50) is comprised by, joined to or coextensive of said wearer facing portion (16).

3. A human waste collection bag according to claims 1 or 2, wherein said stress relief means (50) is provided to relief stress exerted onto the perineal area of the flange (12).

4. A human waste collection bag according to claims 1, 2 or 3, wherein said stress relief means (50) comprises a stretchable material (52).

5. A human waste collection bag according to claim 4, wherein said stretchable material has a strain modulus of less than 1000 grams per percent strain per cm width.

6. A human waste collection bag according to any one of the preceding claims wherein that stress relief means (50) comprises an orifice (54).

7. A human waste collection bag according claim 6 wherein said pouch (11) is partially not joined to said flange (12) such that an orifice (54) is provided.

8. A human waste collection bag according claim 6 wherein said orifice is covered by a curtain (56).

9. A human waste collection bag according claim 7 wherein said curtain is attached to said flange (12).

10. A human waste collection bag according to any one of the preceding claims wherein said stress relief means (50) comprises a portion of the pouch (11) in which extra material (58) is provided between the flange (12) and the distal end of the pouch (11) than in adjacent portions.
